# EUROPÄISCHE PATENTANMELDUNG

(11) **EP 2 883 567 A1**
(43) Veröffentlichungstag der Anmeldung: **17.06.2015**
(21) Anmeldenummer: 13005766.4
(22) Anmeldetag: 11.12.2013
(51) Int. Cl.: A61N 5/10, A61B 6/04, A61G 13/00

(54) **Patientenpositioniereinrichtung und Verfahren zur Positionierung eines Patienten an einer Bestrahlungseinrichtung mittels einer Patientenpositioniereinrichtung**

(71) Anmelder: Buck Engineering & Consulting GmbH, 72764 Reutlingen (DE)
(72) Erfinder: Buck, Matthias, 72764 Reutlingen (DE)
(74) Vertreter: Reinhardt, Annette

(57) **Zusammenfassung**

Für eine Patientenpositioniereinrichtung (1) mit einem Roboterarm (2), der mindestens fünf Rotationsbewegungsachsen (16, 17, 18, 19, 20, 21) aufweist und an dem eine Patientenaufnahme gehalten ist, ist vorgesehen, dass die Patientenpositioniereinrichtung mindestens eine Linearführung (3) umfasst, mittels der der Roboterarm (2) translatorisch verfahrbar ist. Ein Verfahren zur Positionierung eines Patienten an einer Bestrahlungseinrichtung (74) mittels der Patientenpositioniereinrichtung (1) sieht vor, dass Daten übernommen werden, die die Position eines Zielbereichs (71, 72) der Bestrahlungseinrichtung (74) angeben, dass die Position eines zu bestrahlenden Bereichs (85) des Patienten (75) mittels einer bildgebenden Einheit (9) ermittelt wird, und dass die Patientenaufnahme anschließend so bewegt wird, dass der zu bestrahlende Bereich (75) in dem Zielbereich (71, 72) der Bestrahlungseinrichtung (74) angeordnet ist.

## Beschreibung

Die Erfindung betrifft eine Patientenpositioniereinrichtung der im Oberbegriff des Anspruchs 1 angegebenen Gattung und ein Verfahren zur Positionierung eines Patienten an einer Bestrahlungseinrichtung mittels einer Patientenpositioniereinrichtung nach dem Oberbegriff des Anspruchs 14.

Aus der EP 1 985 237 A1 ist eine Patientenpositioniereinrichtung bekannt, die einen Roboterarm besitzt. An dem Roboterarm ist eine Patientenaufnahme gehalten. Mit dem Roboterarm kann die Patientenaufnahme an einer Bestrahlungseinrichtung positioniert werden.

Bei Bestrahlungseinrichtungen, insbesondere Bestrahlungseinrichtungen, die mit Photonen oder Ionen arbeiten, wird der Strahl in einer Strahlvorbereitung so erzeugt, dass der Strahl für eine Behandlung gewünschte Eigenschaften besitzt. Dabei kann beispielsweise die Reichweite oder Energie des Strahls reduziert werden. Der Strahl besitzt einen Zielbereich, in dem der Strahl die für die Behandlung gewünschten Eigenschaften aufweist, beispielsweise eine gewünschte Energie und/oder Streuung des Strahls. Die Lage des Zielbereichs der Bestrahlungseinrichtung ist abhängig von Faktoren und Einrichtungen der Strahlvorbereitung, die behandlungsrelevante Eigenschaften des Strahls beeinflussen. Bekannte Bestrahlungseinrichtungen beeinflussen den Strahl so, dass der Zielbereich im Raum immer eine näherungsweise gleiche Lage hat. Dadurch können Patientenpositioniereinrichtungen mit vergleichsweise begrenzter Reichweite eingesetzt werden. Der Aufbau der Bestrahlungseinrichtung ist dadurch jedoch vergleichsweise komplex.

Der Erfindung liegt die Aufgabe zugrunde, eine Patientenpositioniereinrichtung der gattungsgemäßen Art zu schaffen, die eine große Reichweite bei einfachem Aufbau besitzt. Eine weitere Aufgabe der Erfindung ist es, ein Verfahren zur Positionierung eines Patienten an einer Bestrahlungseinrichtung mittels einer Patientenpositioniereinrichtung anzugeben, das einen einfachen Aufbau der Gesamtanordnung ermöglicht.

Diese Aufgabe wird bezüglich der Patientenpositioniereinrichtung mit den Merkmalen des Anspruchs 1 gelöst. Bezüglich des Verfahrens wird die Aufgabe durch ein Verfahren mit den Merkmalen des Anspruchs 14 gelöst.

Es ist vorgesehen, dass die Patientenpositioniereinrichtung mindestens eine Linearführung umfasst. Mittels der Linearführung ist der Roboterarm translatorisch verfahrbar. Dadurch wird die Reichweite der Patientenpositioniereinrichtung in der Richtung, in der der Roboterarm mittels der Linearführung translatorisch verfahrbar ist, deutlich vergrößert. Es hat sich gezeigt, dass insbesondere für die Positionierung an einer Bestrahlungseinrichtung eine große Reichweite in Längsrichtung des Strahls erforderlich ist, während in Querrichtung hierzu auch geringere Reichweiten ausreichend sein können. Aufgrund der Linearachse kann der benötigte Roboterarm kleiner ausgeführt sein als ein Roboterarm einer Patientenpositioniereinrichtung, bei der die gesamte Beweglichkeit vom Roboterarm bereitgestellt wird. Dadurch ergibt sich trotz der zusätzlichen Linearführung ein insgesamt einfacher Aufbau.

Vorteilhaft umfasst die Linearführung einen Schlitten, an dem der Roboterarm festgelegt ist. Die Linearführung umfasst insbesondere zwei Führungsschienen, die an gegenüberliegenden Längsseiten des Schlittens angeordnet sind. Die Führungsschienen sind dadurch im Raum neben dem Schlitten angeordnet. Dadurch ergibt sich eine geringe Bauhöhe der Linearführung. An den Führungsschienen ist der Schlitten bei seiner Translationsbewegung geführt.

Die Linearführung weist vorteilhaft eine Antriebseinheit auf. Um einen einfachen Aufbau und eine geringe Bauhöhe zu erreichen, ist vorgesehen, dass die Antriebseinheit in Verfahrrichtung des Schlittens vor bzw. hinter dem Schlitten angeordnet ist. Vorteilhaft ist die Patientenpositioniereinrichtung an einer Bestrahlungseinrichtung angeordnet. Die Antriebseinheit ist vorteilhaft an der der Bestrahlungseinrichtung abgewandten Seite des Schlittens angeordnet. Dadurch kann der Schlitten bis nahe an die Bestrahlungseinrichtung herangefahren werden. Dies ist insbesondere dann vorteilhaft, wenn die Bestrahlungseinrichtung eine Gantry umfasst. Dadurch, dass die Antriebseinheit an der der Bestrahlungseinrichtung abgewandten Seite des Schlittens angeordnet ist, kann der Schlitten nahe bis an die Gantry verfahren. Dadurch kann eine ausreichende Reichweite des Roboterarms in das Isozentrum der Gantry, also in den Zielbereich des Strahls, ermöglicht werden.

Ein einfacher Aufbau ergibt sich, wenn die Linearführung einen Rahmen aufweist, an dem der Schlitten geführt ist. Vorteilhaft ist die Höhe des Rahmens an die Höhe der Antriebseinheit angepasst. Die Antriebseinheit weist vorteilhaft eine senkrecht zur Verfahrrichtung gemessene Höhe auf, die etwa 65% bis etwa 110% einer senkrecht zur Verfahrrichtung gemessenen Höhe des Rahmens der Linearführung beträgt. Die Höhe der Antriebseinheit und die Höhe des Rahmens sind dabei in der gleichen Richtung gemessen, die vorteilhaft senkrecht im Raum verläuft. Die Höhe der Antriebseinheit beträgt insbesondere etwa 70% bis etwa 90% der Höhe des Rahmens der Linearführung. Die Antriebseinheit erstreckt sich damit über einen Großteil der Höhe des Rahmens der Linearführung. Die Antriebseinheit kann dabei geringfügig über den Rahmen der Antriebseinheit überstehen. Es kann jedoch auch vorteilhaft sein, dass die Antriebseinheit vollständig innerhalb des Rahmens der Linearführung angeordnet ist und keinen Überstand aufweist. Dadurch, dass die Antriebseinheit vor oder hinter dem Schlitten angeordnet ist, kann auch eine Antriebseinheit, deren Höhe mindestens etwa 65% der Höhe des Rahmens beträgt, weitgehend in dem Rahmen angeordnet werden. Dadurch ergeben sich eine geringe Bauhöhe, ein einfacher Aufbau und eine ansprechende optische Gestaltung.

Vorteilhaft weist die Linearführung mindestens eine Lamellenabdeckung auf, deren freies Ende mit dem Schlitten verbunden ist und die sich zwischen gegenüberliegenden Seitenwänden des Rahmens erstreckt. Die Lammellenabdeckung deckt vorzugsweise die Führungsschienen der Linearführung ab, so dass sich eine geschlossene Außenkontur der Linearführung und ein ansprechendes Äußeres ergeben. Die Lamellenabdeckung kann beispielsweise auch eine Zahnstange, mit der die Antriebseinheit zusammenwirkt und/oder eine oder mehrere Energieketten abdecken. Vorzugsweise sind zwei Lamellenabdeckungen vorgesehen, die in Verfahrrichtung des Schlittens vor und hinter dem Schlitten angeordnet sind. Die Antriebseinheit ist vorzugsweise zwischen dem freien Ende einer Lamellenabdeckung und dem Schlitten angeordnet. Dadurch, dass die Antriebseinheit nicht von der Lamellenabdeckung abgedeckt ist, kann auf einfache Weise eine ausreichende Kühlung der Antriebseinheit gewährleistet werden. Gleichzeitig kann eine Antriebseinheit zum Einsatz kommen, die geringfügig über den Rahmen der Linearführung übersteht.

Vorteilhaft besitzt die Linearführung mindestens eine erste Energiekette zur Versorgung des Roboterarms mit Energie und mindestens eine zweite Energiekette für Signal- und Versorgungsleitungen für die Patientenaufnahme. Die Signalleitungen können dabei Leitungen sein, die mit Sensoren verbunden sind, die eine Kollision der Patientenliege mit Gegenständen im Raum vermeiden. Die Signalleitungen können auch zur Übertragung von Signalen von und zu einer an der Patientenaufnahme angeordneten bildgebenden Einheit dienen. Versorgungsleitungen können beispielsweise Leitungen zur Versorgung des Patienten mit Gasen oder Flüssigkeiten, beispielsweise mit Anästhesiegas oder dgl. sein. Um eine kompakte Anordnung der Energieketten zu ermöglichen, ist vorgesehen, dass die Biegeachse, um die die erste Energiekette gebogen ist, senkrecht zu der Biegeachse steht, um die die zweite Energiekette gebogen ist.

Für eine Patientenaufnahme, an der eine bildgebende Einheit montiert ist, ist vorzugsweise eine dritte Energiekette vorgesehen. Vorzugsweise liegt die Biegeachse der dritten Energiekette parallel zur Biegeachse der zweiten Energiekette. Bei horizontaler Anordnung der Linearachse ist die Biegeachse der ersten Energiekette vorteilhaft horizontal und die Biegeachse der zweiten Energiekette vorteilhaft vertikal angeordnet. Insbesondere ist die Biegeachse der dritten Energiekette vertikal angeordnet.

Vorteilhaft ist die Linearführung deckenmontiert. Dadurch ist der Fußboden im Bereich der Patientenpositioniereinrichtung frei zugänglich. Die Bewegungsfreiheit von medizinischem Personal wird durch die Patientenpositioniereinrichtung nicht eingeschränkt. Dadurch, dass der Roboterarm an der Linearführung geführt ist, kann ein Roboterarm mit geringerer Reichweite, der vergleichsweise klein und leicht aufgebaut ist, zum Einsatz kommen. Durch das vergleichsweise geringe Gewicht des Roboterarms ist die Montage an der Decke vereinfacht.

Ein Verfahren zur Positionierung eines Patienten an einer Bestrahlungseinrichtung mittels einer Patientenpositioniereinrichtung, wobei die Patientenpositioniereinrichtung einen Roboterarm umfasst, der mindestens fünf Rotationsbewegungsachsen aufweist, wobei an dem Roboterarm eine Patientenaufnahme gehalten ist, und wobei die Patientenpositioniereinrichtung mindestens eine Linearführung umfasst, mittels der der Roboterarm translatorisch verfahrbar ist, sieht vor, Daten, die die Position eines Zielbereichs der Bestrahlungseinrichtung angeben, von der Bestrahlungseinrichtung zu übernehmen, die Position eines zu bestrahlenden Bereichs des Patienten mittels einer bildgebenden Einheit zu ermitteln und in einem weiteren Schritt die Patientenaufnahme so zu bewegen, dass der zu bestrahlende Bereich in dem Zielbereich der Bestrahlungseinrichtung angeordnet ist. Die Übernahme der Daten, die die Position des Zielbereichs der Bestrahlungseinrichtung angeben und das Ermitteln der Position eines zu bestrahlenden Bereichs des Patienten können dabei gleichzeitig oder in beliebiger Reihenfolge erfolgen. Diese beiden Schritte können auch in deutlichem zeitlichen Abstand zueinander erfolgen.

Dadurch, dass die Patientenpositioniereinrichtung eine Linearführung umfasst, besitzt die Patientenpositioniereinrichtung eine sehr große Reichweite. Dadurch ist eine Anpassung der Lage des Zielbereichs der Bestrahlungseinrichtung durch die Bestrahlungseinrichtung im Makrobereich nicht erforderlich. Dadurch kann eine Bestrahlungseinrichtung mit einfachem Aufbau zum Einsatz kommen. Über die Linearführung kann die Position des Patienten auf einfache Weise an die tatsächliche Lage des Zielbereichs der Bestrahlungseinrichtung angepasst werden. Der Gesamtaufbau der Anlage vereinfacht sich dadurch. Durch die große Reichweite der Patientenpositioniereinrichtung ist es möglich, den Patienten sehr nah an eine Austrittsöffnung des Strahls heranzufahren. Dadurch kann eine geringe Aufweitung des Strahls in dem zu bestrahlenden Bereich und dadurch eine gezielte Bestrahlung auch kleiner zu bestrahlender Bereiche erreicht werden.

Vorteilhaft ist die Bestrahlungseinrichtung ortsfest angeordnet, und die Verfahrrichtung der Linearführung ist parallel zur Richtung des Strahls der Bestrahlungseinrichtung ausgerichtet. Dadurch kann der Roboterarm mit der Patientenaufnahme über die Linearführung in Richtung des Strahls bewegt und dadurch der zu bestrahlende Bereich des Patienten in den Zielbereich der Bestrahlungseinrichtung gebracht werden. Die Linearführung ermöglicht, den Patienten über die gesamte Lage des Bereichs, in dem der von Strahlenart und Strahlintensität abhängige Zielbereich liegen kann, so zu bewegen, dass der zu bestrahlende Bereich in dem Zielbereich liegt.

Ein Ausführungsbeispiel der Erfindung wird im Folgenden anhand der Zeichnung erläutert. Es zeigen:
- Fig. 1: eine Seitenansicht einer Patientenpositioniereinrichtung an einer Bestrahlungseinrichtung, wobei die Patientenaufnahme nicht gezeigt ist,
- Fig. 2: eine Seitenansicht der Patientenpositioniereinrichtung in Richtung des Pfeils II in Fig. 1,
- Fig. 3: eine perspektivische Darstellung der Patientenpositioniereinrichtung aus den Figuren 1 und 2 mit daran angeordneter Patientenaufnahme,
- Fig. 4: eine Seitenansicht auf die Patientenpositioniereinrichtung aus Fig. 3,
- Fig. 5: eine Seitenansicht auf die Patientenpositioniereinrichtung in Richtung des Pfeils V in Fig. 4,
- Fig. 6: eine Seitenansicht der Linearführung der Patientenpositioniereinrichtung,
- Fig. 7: einen Schnitt entlang der Linie VII-VII in Fig. 6,
- Fig. 8: einen Schnitt entlang der Linie VIII-VIII in Fig. 6,
- Fig. 9: eine Seitenansicht in Richtung des Pfeils IX in Fig. 6,
- Fig. 10: einen Schnitt entlang der Linie X-X in Fig. 9,
- Fig. 11: eine Seitenansicht der Linearführung in einer weiteren Position des Schlittens,
- Fig. 12: einen Schnitt entlang der Linie XII-XII in Fig. 11,
- Fig. 13: eine Draufsicht in Richtung des Pfeils XIII in Fig. 11,
- Fig. 14: eine Ansicht von unten in Richtung des Pfeils XIV in Fig. 11, wobei die Lamellenabdeckungen nicht gezeigt sind,
- Fig. 15: einen Schnitt entlang der Linie XV-XV in Fig. 13,
- Fig. 16: einen Schnitt entlang der Linie XVI-XVI in Fig. 13,
- Fig. 17: eine Ansicht von unten entsprechend Fig. 14, wobei die Lamellenabdeckungen der Linearführung gezeigt sind,
- Fig. 18: eine Seitenansicht der Patientenpositioniereinrichtung an einer weiteren Bestrahlungseinrichtung,
- Fig. 19 und Fig. 20: die Anordnung aus Fig. 18 in unterschiedlichen Positionen des Roboterarms an der Linearführung.

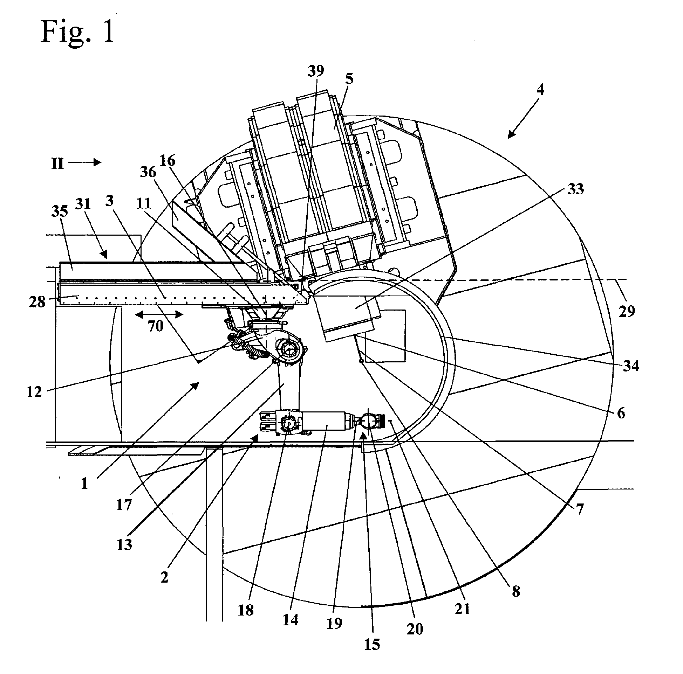

Fig. 1 zeigt eine Patientenpositioniereinrichtung 1 an einer Bestrahlungseinrichtung 4. Im Ausführungsbeispiel umfasst die Bestrahlungseinrichtung 4 eine Gantry 5, die um ein Isozentrum 8 drehbar ist, so dass der Patient aus unterschiedlichen Richtungen bestrahlt werden kann. Die Bestrahlungseinrichtung 4 erzeugt einen Strahl 7, der an einer Austrittsöffnung 6 aus einer Nozzle 33 austritt. Die Nozzle 33 weist seitliche Führungen 34 auf, entlang derer sich die Nozzle 33 bewegt.

Die Patientenpositioniereinrichtung 1 dient dazu, einen Patienten im Isozentrum 8 so zu positionieren, dass ein zu bestrahlender Bereich des Patienten im Isozentrum 8 liegt. Im Ausführungsbeispiel erzeugt die Bestrahlungseinrichtung 4 einen Strahl 7 aus Protonen, Neutronen oder Ionen, vorzugsweise schweren Ionen. Es kann auch eine Bestrahlungseinrichtung 4 vorgesehen sein, die einen Photonenstrahl wie einen Röntgen- oder Gammastrahl erzeugt. Auch eine andere Art von Strahlung kann vorgesehen sein. Die Bestrahlungseinrichtung 4 kann zur Diagnose und/oder zur Therapie, beispielsweise zur Tumorbestrahlung, dienen.

Die Patientenpositioniereinrichtung 1 umfasst einen Roboterarm 2, der an einer Linearführung 3 gehalten und mittels der Linearführung 3 translatorisch verfahrbar ist. Die Linearführung 3 besitzt einen Rahmen 28, der an einer Raumdecke 29 fixiert ist. Die Patientenpositioniereinrichtung 1 ist dadurch an der Raumdecke 29 gehalten und besitzt im Betrieb üblicherweise keinen Kontakt zum Boden. Dadurch wird eine gute Zugänglichkeit erreicht und Behinderungen des medizinischen Personals durch die Patientenpositioniereinrichtung 1 werden vermieden. Der Rahmen 28 ist mittels einer Befestigungsstruktur 31 in der Raumdecke 29 verankert. Die Befestigungsstruktur 31 besitzt einen Längsträger 35 sowie an der der Gantry 5 zugewandten Seite geneigte Träger 36. Die geneigten Träger 36 sind so ausgerichtet, dass die Gantry 5 bis unmittelbar an die geneigten Träger 36 verfahren kann. Wie Fig. 1 zeigt, besitzt der Rahmen 28 an der der Gantry 5 zugewandten Seite eine Abschrägung 39, die etwa im gleichen Winkel verläuft wie die geneigten Träger 36. Dadurch kann der Roboterarm 2 bis nahe an den Schwenkbereich der Gantry 5 verfahren werden.

Der Roboterarm 2 umfasst ein Grundgestell 11, an dem ein Karussell 12 um eine erste Rotationsbewegungsachse 16 schwenkbar gelagert ist. Die erste Rotationsbewegungsachse 16 ist im Ausführungsbeispiel senkrecht angeordnet. Am Karussell 12 ist eine Schwinge 13 um eine zweite Rotationsbewegungsachse 17 schwenkbar gelagert. Die zweite Rotationsbewegungsachse 17 ist im Ausführungsbeispiel horizontal und senkrecht zu einer Verfahrrichtung 70 der Linearführung 3 angeordnet. An der Schwinge 13 ist ein Arm 14 um eine dritte Rotationsbewegungsachse 18 schwenkbar gelagert. Die dritte Rotationsbewegungsachse 18 ist parallel zur zweiten Rotationsbewegungsachse 17 angeordnet. Am Arm 14 ist ein Handabschnitt 15 angeordnet, der gegenüber dem Arm 14 um eine vierte Rotationsbewegungsachse 19 schwenkbar ist. Die vierte Rotationsbewegungsachse 19 verläuft in Längsrichtung des Arms 14. Der Handabschnitt 15 besitzt außerdem eine fünfte Rotationsbewegungsachse 20 sowie eine sechste Rotationsbewegungsachse 21. Die fünfte Rotationsbewegungsachse 20 liegt senkrecht zur vierten Rotationsbewegungsachse 19. Die sechste Rotationsbewegungsachse 21 liegt senkrecht zur fünften Rotationsbewegungsachse 20. Eine am Handabschnitt 15 zu fixierende Patientenaufnahme, beispielsweise eine Patientenliege oder ein Patientenstuhl, ist in Fig. 1 nicht gezeigt.

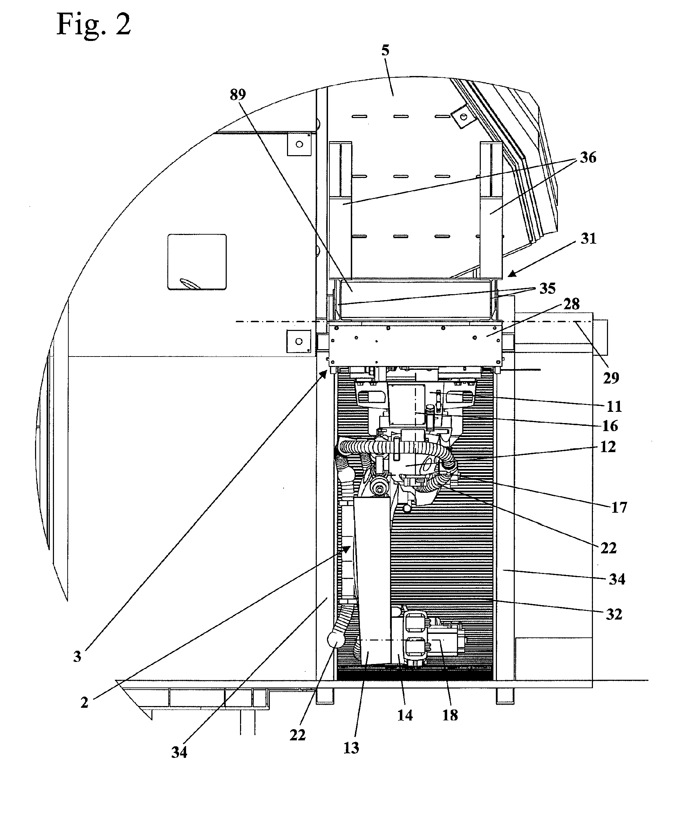

In Fig. 2 ist die Anordnung der Rotationsbewegungsachsen 16, 17 und 18 ebenfalls gezeigt. Wie Fig. 2 auch zeigt, sind die einzelnen Abschnitte des Roboterarms 2 über Leitungsführungen 22 miteinander verbunden, in denen Energie-, Sensor- und Versorgungsleitungen geführt sind. Wie Fig. 2 auch zeigt, ist an der Gantry 5 benachbart zur in Fig. 2 nicht sichtbaren Nozzle 33 eine Lamellenabdeckung 32 angeordnet. Die Lamellenabdeckung 32 ist nach Art eines Rollladens aufgebaut und an der Nozzle 33 fixiert. Vorzugsweise sind zwei Lamellenabdeckungen 32 beidseitig der Nozzle 33 vorgesehen. Die Lamellenabdeckungen 32 können mit einem eigenen Antrieb versehen sein, der das Auf- und Abrollen der Lamellenabdeckungen 32 gewährleistet. In Fig. 2 ist auch der Längsträger 35 gezeigt. Im Ausführungsbeispiel sind zwei Längsträger 35 vorgesehen, die über einen oder mehrere Querträger 89 miteinander verbunden sind.

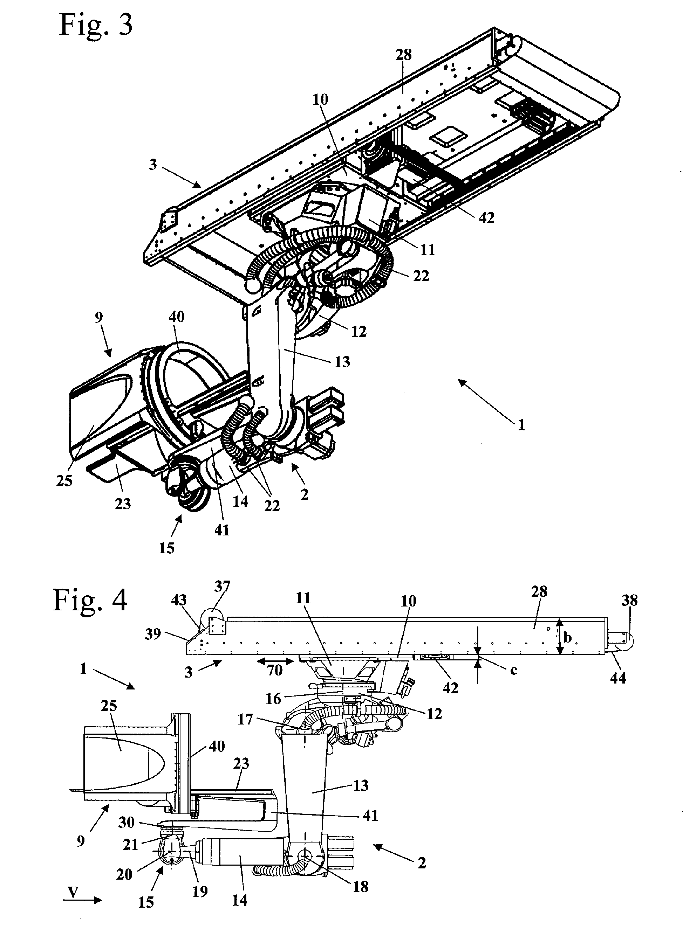

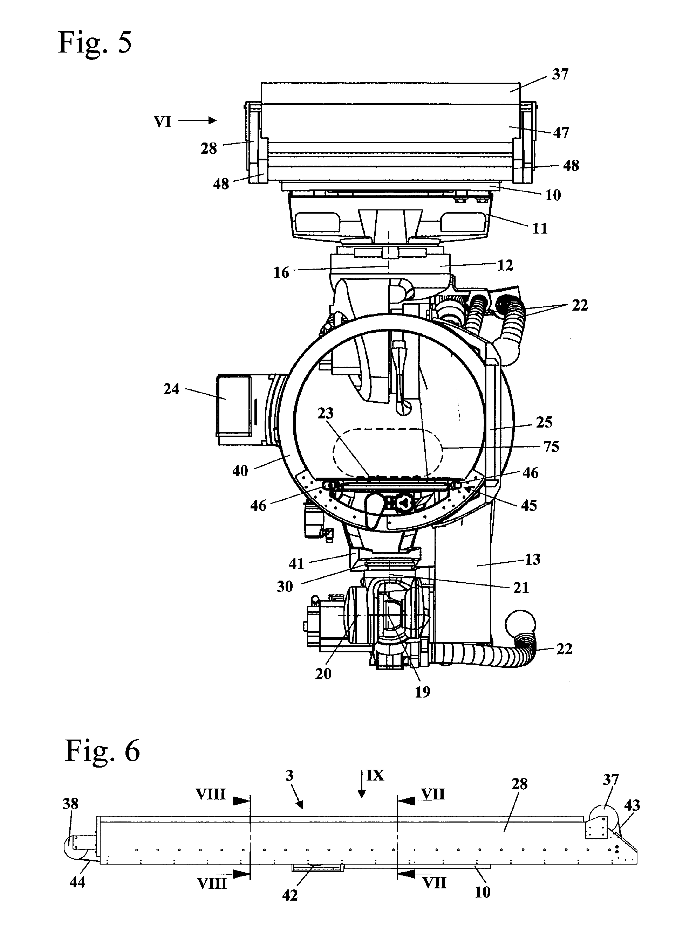

Fig. 3 zeigt die Patientenpositioniereinrichtung 1 mit am Roboterarm 2 angeordneter Patientenliege 23. An der Patientenliege 23 ist eine bildgebende Einheit 9 längsverfahrbar gehalten. Die bildgebende Einheit 9 umfasst eine in Fig. 5 gezeigte Strahlenquelle 24 sowie einen Empfängerschirm 25. Die Strahlenquelle 24 und der Empfängerschirm 25 sind an einem Lagerring 40 um die Patientenliege 23 drehbar gehalten. Die Strahlenquelle 24 kann beispielsweise eine Röntgenquelle sein. Wie Fig. 3 zeigt, ist die Patentenliege 23 am Handabschnitt 15 des Roboterarms 2 über einen Bügel 41 gehalten. Der Bügel 41 ist in Seitenansicht etwa C-förmig ausgebildet und ermöglicht, dass die bildgebende Einheit 9 über einen Großteil der Länge der Patientenliege 23 verfahrbar ist. Wie die Figuren 4 und 5 zeigen, besitzt der Handabschnitt 15 einen Befestigungsflansch 30, an dem der Bügel 41 fixiert ist. Der Bügel 41 kann beispielsweise am Befestigungsflansch 30 verschraubt oder aufgelegt sein. Wie Fig. 3 auch zeigt, besitzt die Linearführung 3 eine Antriebseinheit 42. Die Antriebseinheit 42 ist an einem Schlitten 10 der Linearführung 3 festgelegt. An dem Schlitten 10 ist das Grundgestell 11 des Roboterarms 2 fixiert.

Wie Fig. 4 zeigt, ragt die Antriebseinheit 42 aus dem Rahmen 28 nach unten in Richtung auf den Roboterarm 2 hinaus. Die Antriebseinheit 42 besitzt gegenüber dem Rahmen 28 einen Überstand c. Vorteilhaft beträgt der Überstand c einen Bruchteil einer Höhe b des Rahmens 28. Der Überstand c ist vorteilhaft kleiner als 25% der Höhe b. Die Höhe b und der Überstand c sind senkrecht zur Verfahrrichtung 70 und zur Oberseite des Rahmens 28 bzw. zur Raumdecke 29 gemessen.

In Fig. 4 sind auch Lamellenabdeckungen 43 und 44 sichtbar, die in Verfahrrichtung 70 vor und hinter dem Schlitten 10 angeordnet sind. An der im Betrieb der Gantry 5 zugewandten Seite verläuft die erste Lamellenabdeckung 43, die an einer Walze 37 aufgerollt ist. Die Walze 37 ist im oberen Bereich des Rahmens 28 angeordnet und im Bereich der Abschrägung 39 von der Gantry 5 weg versetzt angeordnet, so dass ausreichend Bauraum für die Gantry 5 vorhanden ist. An der gegenüberliegenden, der Gantry 5 abgewandten Seite ist eine zweite Lamellenabdeckung 44 angeordnet, die an einer Walze 38 aufgerollt ist. Die Antriebseinheit 42 ist an der der Gantry 5 abgewandten Seite des Schlittens 10 angeordnet. Dadurch kann der Roboterarm 2 bis nahe an die Gantry 5 gefahren werden. Die Walzen 37 und 38 sind vorteilhaft in Aufrollrichtung federbelastet, beispielsweise durch eine Spiralfeder. Es kann jedoch auch vorteilhaft sein, einen Antrieb für die Walzen 37 und 38 vorzusehen.

In Fig. 5 ist schematisch ein auf der Patientenliege 23 angeordneter Patient 75 gezeigt. Der Lagerring 40 ist mittels einer Längsführung 45 längsverschiebbar an der Patientenliege 23 gehalten. Die Längsführung 45 umfasst zwei Schienen 46, an denen der Lagerring 40 geführt ist. Wie Fig. 5 auch zeigt, besitzt der Rahmen 28 Seitenführungen 48 für die Lamellenabdeckungen 43 und 44. Die Lamellenabdeckungen 43 und 44 sind in Fig. 5 nicht gezeigt. In Fig. 5 ist ein Leitblech 47 sichtbar, an dem die Lamellenabdeckung 43 geführt ist. Ein entsprechendes Leitblech 47 kann auch für die zweite Lamellenabdeckung 44 vorgesehen sein.

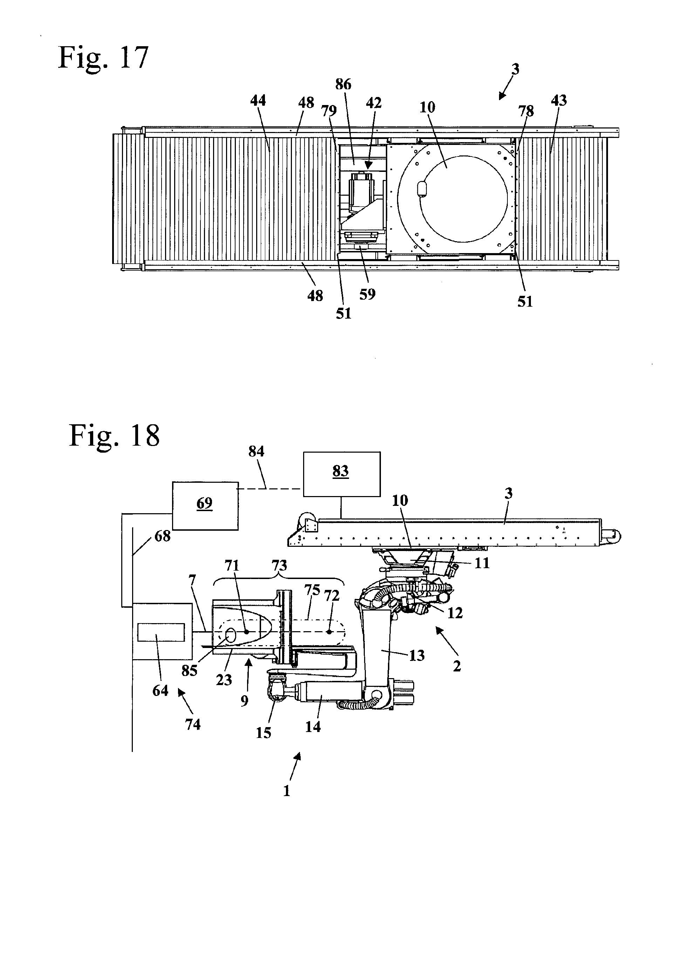

Die Lamellenabdeckungen 43 und 44 sind in Fig. 17 gezeigt. Wie Fig. 17 zeigt, besitzt die Lamellenabdeckung 43 ein freies Ende 78, das am Schlitten 10 festgelegt ist. Die Lamellenabdeckung 44 besitzt ein freies Ende 79, das benachbart zur Antriebseinheit 42 angeordnet und über die Antriebseinheit 42 sowie einen Befestigungssteg 86 mit dem Schlitten 10 verbunden ist. Die freien Enden 78, 79 der Lamellenabdeckungen 43 und 44 sind dabei an Befestigungsschienen 51 fixiert, die am Schlitten 10 gehalten sind.

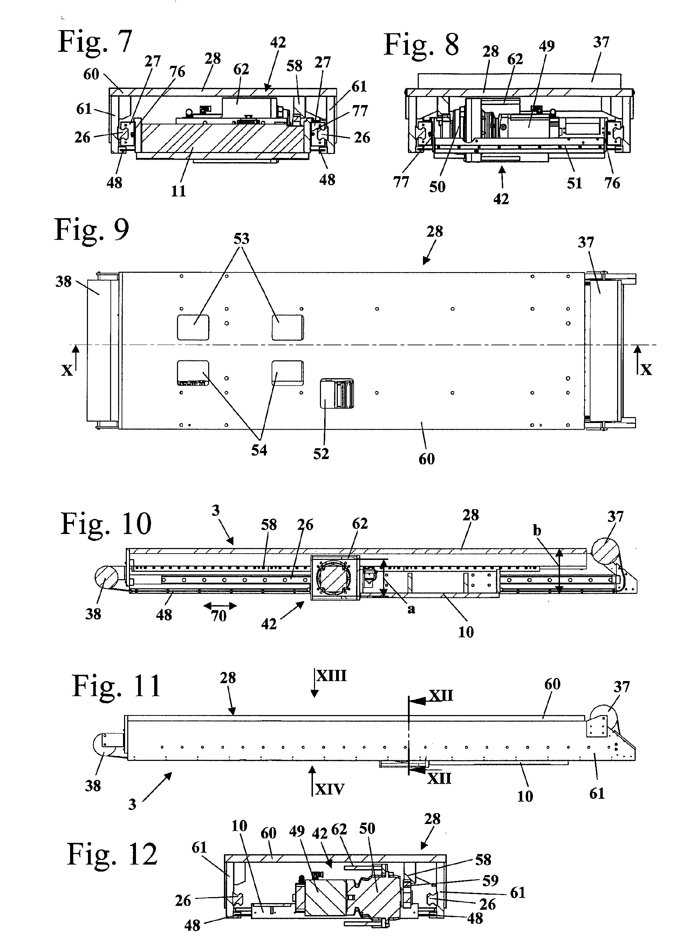

Die Figuren 6 bis 10 zeigen die Linearführung 3 in einer Stellung, in der der Schlitten 10 in einer mittleren Position angeordnet ist. Wie Fig. 7 zeigt, besitzt der Rahmen 28 eine Deckplatte 60, die benachbart zur Raumdecke 29 (Fig. 1) angeordnet ist, sowie Seitenwände 61, die sich in Längsrichtung der Linearführung 3, also parallel zur Verfahrrichtung 70, und senkrecht zur Deckplatte 60 erstrecken. An den Seitenwänden 61 ist jeweils eine Führungsschiene 26 angeordnet, die als Profilschiene ausgebildet ist. Der Schlitten 10 besitzt Führungselemente 27, die die Führungsschienen 26 übergreifen und die an den Führungsschienen 26 in Verfahrrichtung 70 geführt sind. Der Schlitten 10 besitzt einander gegenüberliegende Längsseiten 76 und 77, die parallel zu den Seitenwänden 61 des Rahmens 28 verlaufen. Im Ausführungsbeispiel sind an jeder Längsseite 76, 77 des Schlittens 10 zwei Führungselemente 27 angeordnet. In Fig. 7 ist auch die Anordnung der Seitenführungen 48 für die Lamellenabdeckungen 43 und 44 gezeigt. Die Seitenführungen 48 sind an den Seitenwänden 61 jeweils an der dem Schlitten 10 zugewandten Innenseite benachbart zu dem der Deckplatte 60 abgewandten freien Ende der Seitenwände 61 angeordnet. Im Ausführungsbeispiel sind die Führungselemente 27 unmittelbar oberhalb der Seitenführungen 48 geführt. Durch die Anordnung der Führungsschienen 26 und Führungselemente 27 an den gegenüberliegenden Längsseiten 76, 77 des Schlittens 10 ergibt sich eine niedrige Bauhöhe der Gesamtanordnung.

Wie die Figuren 7 und 8 zeigen, ist die Antriebseinheit 42 über einen Befestigungsflansch 62 am Schlitten 10 fixiert. Die Antriebseinheit 42 umfasst einen Antriebsmotor 49 sowie ein Getriebe 50. Wie insbesondere Fig. 12 zeigt, besitzt die Antriebseinheit 42 ein Antriebsritzel 59, das mit einer am Rahmen 28 gehaltenen Zahnstange 58 kämmt und dadurch den Schlitten 10 in Verfahrrichtung 70 (Fig. 10) bewegt.

Wie Fig. 10 zeigt, besitzt die Antriebseinheit 42 eine Höhe a, die senkrecht zur Verfahrrichtung 70 gemessen ist. Die Höhe a ist parallel zur Höhe b des Rahmens 28 gemessen. Die Höhe a entspricht im Ausführungsbeispiel der Höhe des Getriebes 50. Der Befestigungsflansch 62 ist nicht Teil der Antriebseinheit 42, so dass die Höhe des Befestigungsflanschs 62 nicht berücksichtigt ist. Wie Fig. 10 zeigt, ist die Höhe a der Antriebseinheit 42 geringfügig kleiner als die Höhe b des Rahmens 28. Die Höhe a beträgt vorteilhaft etwa 65% bis etwa 110%, insbesondere etwa 70% bis etwa 90% der Höhe b des Rahmens 28. Die Antriebseinheit 42 ist dabei so angeordnet, dass der in Fig. 4 gezeigte Überstand c möglichst gering ist.

Wie Fig. 10 zeigt, erstreckt sich die Führungsschiene 26 über näherungsweise die gesamte Länge des Rahmens 28. Auch die Zahnstange 58 erstreckt sich über einen Großteil der Länge des Rahmens 28. Die Länge des Rahmens 28 ist dabei in Verfahrrichtung 70 gemessen.

Wie Fig. 9 zeigt, besitzt die Deckplatte 60 des Rahmens 28 Wartungsöffnungen 52, 53 und 54. Es sind jeweils zwei Wartungsöffnungen 53 und 54 vorgesehen.

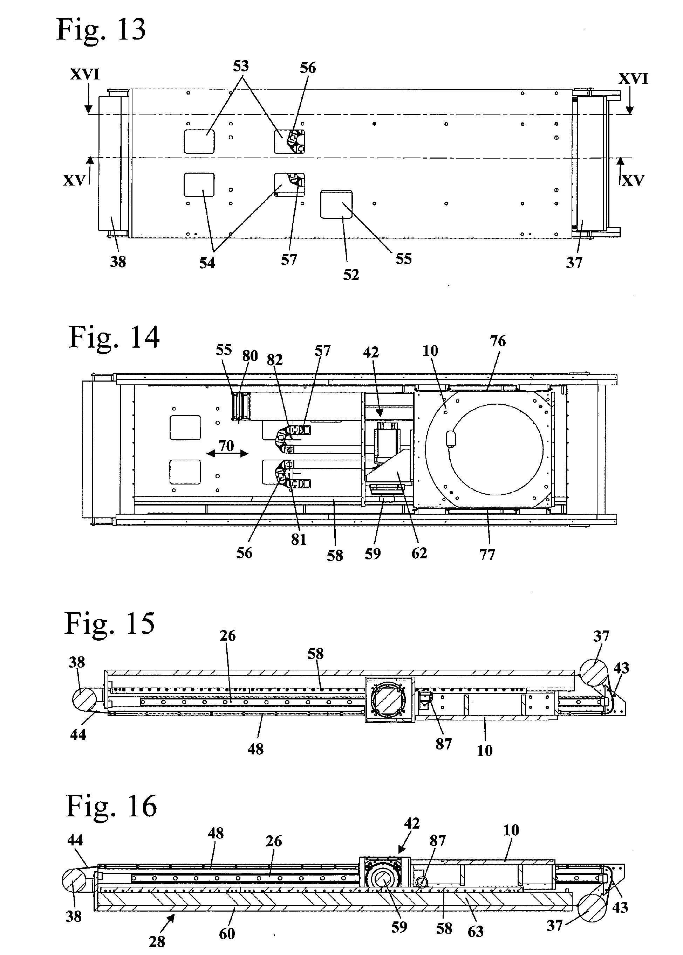

Die Figuren 11 bis 16 zeigen den Schlitten 10 nahe der der Gantry 5 zugewandten Endlage. In den Figuren 13 und 14 sind Energieketten 55, 56 und 57 der Linearführung 3 gezeigt. Eine erste Energiekette 55 verläuft im Bereich der ersten Wartungsöffnung 52, so dass die erste Energiekette 55 durch die erste Wartungsöffnung 52 gewartet werden kann. Die erste Energiekette 55 besitzt eine Biegeachse 80, die im Ausführungsbeispiel horizontal und senkrecht zur Verfahrrichtung 70 verläuft. Die erste Energiekette 55 führt Leitungen zur Zufuhr von Energie zum Roboterarm 2. Die zweite Energiekette 56 besitzt eine Biegeachse 81, die senkrecht zur Biegeachse 80 steht. Die Biegeachse 81 ist vorteilhaft senkrecht im Raum angeordnet. Die zweite Energiekette 56 dient vorteilhaft zur Führung von Signal- und Versorgungsleitungen für die Patientenliege 23. Dies können beispielsweise Signalleitungen für ein System, das eine Kollision der Patientenliege 23 mit anderen Elementen vermeidet, sein. Auch Versorgungsleitungen für den Patienten wie beispielsweise Leitungen zur Zufuhr von Gas oder Flüssigkeiten zum Patienten, können vorgesehen sein. Über die Versorgungsleitungen kann der Patient beispielsweise während der Bestrahlung mit Anästhesiegas versorgt werden.

Vorteilhaft ist eine dritte Energiekette 57 vorgesehen, deren Biegeachse 82 parallel zur Biegeachse 81 der zweiten Energiekette 56 angeordnet ist. Die beiden Energieketten 56 und 57 sind vorteilhaft spiegelsymmetrisch zueinander angeordnet. Die dritte Energiekette 57 ist zwischen der ersten Energiekette 55 und der zweiten Energiekette 56 angeordnet. Die dritte Energiekette 57 dient vorteilhaft zur Führung von Energie- und Signalleitungen für die bildgebende Einheit 9. Dadurch, dass nicht alle Biegeachsen der Energieketten 55, 56 und 57 in der gleichen Richtung ausgerichtet sind, ergibt sich ein kompakter Aufbau. Es wird nur ein geringer Bauraum für die Energieketten 55, 56 und 57 benötigt.

Wie Fig. 16 zeigt, ist die Zahnstange 58 über eine Längsstrebe 63 an der Deckplatte 60 des Rahmens 28 fixiert. Wie die Figuren 15 und 16 zeigen, ist am Schlitten 10 ein Zahnrad 87 festgelegt, das ebenfalls mit der Zahnstange 58 kämmt. Dadurch wird eine gute Führung des Schlittens 10 sichergestellt.

Die Figuren 18 bis 20 zeigen ein Ausführungsbeispiel, bei dem die Patientenpositioniereinrichtung 1 an einer Bestrahlungseinrichtung 74 angeordnet ist. Die Bestrahlungseinrichtung 74 ist vorteilhaft eine ortsfest angeordnete Bestrahlungseinrichtung. Die Bestrahlungseinrichtung 74 ist an einer Wand 68 fixiert. Die Bestrahlungseinrichtung 74 umfasst eine Strahlvorbereitung 64, die beispielsweise ein Streusystem (scattering system), einen Reichweitenmodulator (range modulator), einen Reichweitenverschieber (range shifter) und/oder einen Kollimator umfassen kann. Es können auch weitere oder andere Einrichtung zur Veränderung und Fokussierung des Strahls 7 vorgesehen sein. In Fig. 18 sind schematisch und exemplarisch zwei Zielbereiche 71 und 72 eingezeichnet. Im Ausführungsbeispiel können die Zielbereiche der Bestrahlungseinrichtung 74 in einem mit gestrichelter Linie angedeuteten Bereich 73 liegen. Die Zielbereiche 71, 72 liegen dabei auf der Achse des Strahls 7, wobei der Strahl 7 auch seitlich geringfügig abgelenkt werden kann. Die Patientenpositioniereinrichtung 1 ist so angeordnet, dass die Verfahrrichtung 70 etwa parallel zum Strahl 7 liegt. Durch translatorisches Verfahren des Roboterarms 2 an der Linearführung 3 kann die Lage des Patienten 75 gegenüber dem Zielbereich 71, 72 verändert werden. Im Ausführungsbeispiel ist schematisch ein zu bestrahlender Bereich 85 des Patienten 75 eingezeichnet. Die unterschiedlichen Zielbereiche 71 und 72 können sich beispielsweise dadurch ergeben, dass je nach Bestrahlung unterschiedliche Eigenschaften des Strahls 7 in dem zu bestrahlenden Bereich 85 des Patienten gewünscht werden.

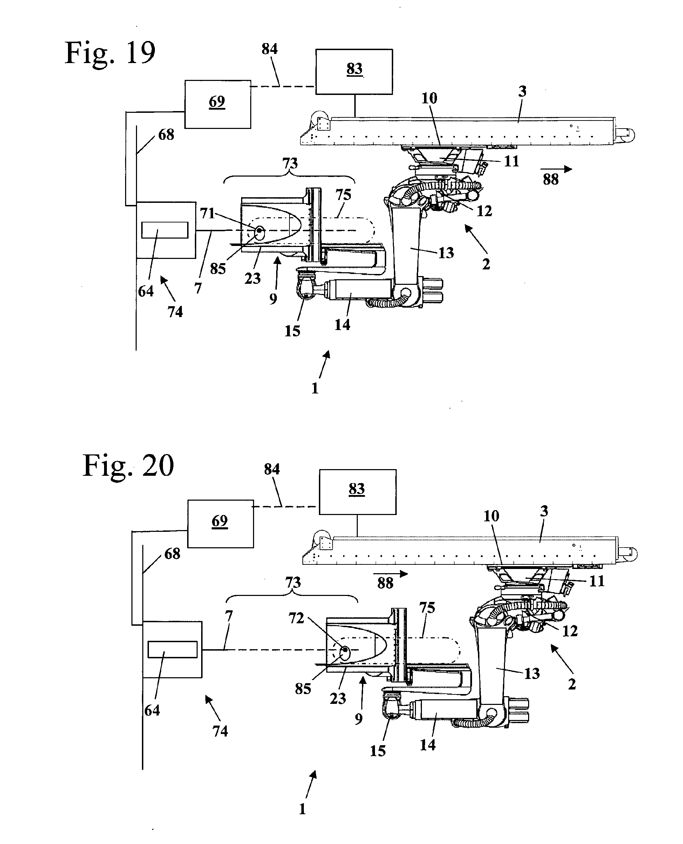

Die Bestrahlungseinrichtung 74 besitzt eine Steuereinrichtung 69, die über eine Datenverbindung 84 mit einer Steuereinrichtung 83 der Patientenpositioniereinrichtung 1 kommunizieren kann. Die Steuereinrichtung 69 übermittelt an die Steuereinrichtung 83 Daten, die die Lage des Zielbereichs 71, 72 für die für den Patienten 75 vorgesehene Bestrahlung innerhalb des Bereichs 73 angeben. Um einen Patienten 75 an der Bestrahlungseinrichtung 74 zu positionieren, ist vorgesehen, dass die Steuereinrichtung 83 von der Steuereinrichtung 69 Daten übernimmt, die die Lage des Zielbereichs 71, 72 für die für diesen Patienten vorgesehene Bestrahlung angeben. Die Patientenpositioniereinrichtung 1 ermittelt außerdem die Position eines zu bestrahlenden Bereichs 85 des Patienten 75 mittels der bildgebenden Einheit 9. Die bildgebende Einheit 9 kann dabei, wie im Ausführungsbeispiel gezeigt, an der Patientenliege 23 gehalten sein. Es kann jedoch auch eine separate bildgebende Einheit 9 vorgesehen sein, die mit der Steuereinrichtung 83 kommuniziert. Nachdem sowohl die Lage des Zielbereichs 71, 72 als auch die Lage des zu bestrahlenden Bereichs 85 des Patienten 75 bekannt sind, ist vorgesehen, dass die Patientenpositioniereinrichtung 1 den Patienten so bewegt, dass sich der zu bestrahlende Bereich 85 in dem Zielbereich 71, 72 der Bestrahlungseinrichtung 74 befindet. Dies ist in Fig. 19 für den ersten Zielbereich 71 gezeigt. Um den zu bestrahlenden Bereich 85 in den Zielbereich 71 zu bewegen, führt die Patientenpositioniereinrichtung 1 im Ausführungsbeispiel eine Bewegung in Richtung des Pfeils 88 in Fig. 19 aus. Diese Bewegung wird vorzugsweise durch eine Bewegung des Schlittens 10 der Linearführung 3 gegenüber dem Rahmen 28 ausgeführt.

Um den Patienten 75 in die in Fig. 20 gezeigte Lage zu bringen, in der der zu bestrahlende Bereich 85 im zweiten Zielbereich 72 liegt, wird der Schlitten 10 um einen deutlich größeren Weg in Richtung des Pfeils 88 bewegt. Es kann jedoch auch vorgesehen sein, dass die Bewegung des Patienten 75 durch eine kombinierte Bewegung von Linearführung 3 und Roboterarm 2 oder ausschließlich durch eine Bewegung des Roboterarms 2 erfolgt. Aufgrund der Linearführung 3 besitzt die Patientenpositioniereinrichtung 1 eine ausreichend große Reichweite, um alle im Bereich 73 liegenden Zielbereiche 71, 72 mit allen Bereichen des Körpers eines Patienten 75 anzufahren. Gleichzeitig ist der Roboterarm 2 vergleichsweise klein und dadurch auch vergleichsweise leicht ausgebildet.

In den Ausführungsbeispielen ist ein Roboterarm 2 mit sechs Rotationsbewegungsachsen gezeigt. Werden weniger Freiheitsgrade der Bewegung benötigt, so kann auch ein Roboterarm mit fünf Rotationsbewegungsachsen ausreichend sein. Es kann auch ein Roboterarm mit mehr als sechs Rotationsbewegungsachsen und/oder zusätzlichen translatorischen Bewegungsachsen vorteilhaft sein.

Im Ausführungsbeispiel ist eine Patientenliege 23 gezeigt, an der eine bildgebende Einheit 9 gehalten ist. Es kann jedoch auch eine Patientenaufnahme ohne bildgebende Einheit vorgesehen sein. Vorzugsweise ist dann eine separate bildgebende Einheit vorgesehen. Im Ausführungsbeispiel ist die Patientenaufnahme eine Patientenliege 23. Auch andere Arten der Patientenaufnahme, beispielsweise ein Stuhl oder dgl., können jedoch vorgesehen sein.

Um eine möglichst geringe Streuung des Strahls 7 zu erreichen, ist es vorteilhaft, den zu bestrahlenden Bereich 85 des Patienten 75 möglichst nah an der Austrittsöffnung 6 des Strahls 7 zu positionieren. Insbesondere bei einer Bestrahlung des Kopfes kann eine seitliche Bestrahlung vorteilhaft sein, bei der der Kopf des Patienten in geringem Abstand zur Austrittsöffnung 6 angeordnet ist. Eine solche Positionierung ist mit der Patientenpositioniereinrichtung 1 einfach möglich.

## Patentansprüche

1. Patientenpositioniereinrichtung mit einem Roboterarm (2), der mindestens fünf Rotationsbewegungsachsen (16, 17, 18, 19, 20, 21) aufweist, wobei an dem Roboterarm eine Patientenaufnahme gehalten ist,
**dadurch gekennzeichnet, dass** die Patientenpositioniereinrichtung (1) mindestens eine Linearführung (3) umfasst, mittels der der Roboterarm (2) translatorisch verfahrbar ist.

2. Patientenpositioniereinrichtung nach Anspruch 1,
**dadurch gekennzeichnet, dass** die Linearführung (3) einen Schlitten (10) umfasst, an dem der Roboterarm (2) festgelegt ist.

3. Patientenpositioniereinrichtung nach Anspruch 2,
**dadurch gekennzeichnet, dass** die Linearführung (3) zwei Führungsschienen (26) umfasst, die an gegenüberliegenden Längsseiten (76, 77) des Schlittens (10) angeordnet sind.

4. Patientenpositioniereinrichtung nach Anspruch 2 oder 3,
**dadurch gekennzeichnet, dass** die Linearführung (3) eine Antriebseinheit (42) aufweist, wobei die Antriebseinheit (42) in Verfahrrichtung (70) vor bzw. hinter dem Schlitten (10) angeordnet ist.

5. Patientenpositioniereinrichtung nach Anspruch 4,
**dadurch gekennzeichnet, dass** die Patientenpositioniereinrichtung (1) an einer Bestrahlungseinrichtung (4, 74) angeordnet ist, und dass die Antriebseinheit (42) an der der Bestrahlungseinrichtung (4, 74) abgewandten Seite des Schlittens (10) angeordnet ist.

6. Patientenpositioniereinrichtung nach Anspruch 4 oder 5,
**dadurch gekennzeichnet, dass** die Linearführung (3) einen Rahmen (28) aufweist, an dem der Schlitten (10) geführt ist.

7. Patientenpositioniereinrichtung nach Anspruch 6,
**dadurch gekennzeichnet, dass** die Antriebseinheit (42) eine senkrecht zur Verfahrrichtung (70) gemessene Höhe (a) aufweist, die etwa 65% bis etwa 110% einer senkrecht zur Verfahrrichtung (70) gemessenen Höhe (b) des Rahmens (28) der Linearführung (3) beträgt.

8. Patientenpositioniereinrichtung nach Anspruch 6 oder 7,
**dadurch gekennzeichnet, dass** die Linearführung (3) mindestens eine Lamellenabdeckung (43, 44) aufweist, deren freies Ende (78, 79) mit dem Schlitten (10) verbunden ist und die sich zwischen gegenüberliegenden Seitenwänden (61) des Rahmens (28) erstreckt.

9. Patientenpositioniereinrichtung nach Anspruch 8,
**dadurch gekennzeichnet, dass** zwei Lamellenabdeckungen (43, 44) vorgesehen sind, die in Verfahrrichtung (70) des Schlittens (10) vor und hinter dem Schlitten (10) angeordnet sind.

10. Patientenpositioniereinrichtung nach Anspruch 8 oder 9,
**dadurch gekennzeichnet, dass** die Antriebseinheit (42) zwischen dem freien Ende (79) einer Lamellenabdeckung (44) und dem Schlitten (10) angeordnet ist.

11. Patientenpositioniereinrichtung nach einem der Ansprüche 1 bis 10,
**dadurch gekennzeichnet, dass** die Linearführung (3) mindestens eine erste Energiekette (55) zur Versorgung des Roboterarms (2) mit Energie und mindestens eine zweite Energiekette (56) für Signal- und Versorgungsleitungen für die Patientenaufnahme besitzt.

12. Patientenpositioniereinrichtung nach Anspruch 11,
**dadurch gekennzeichnet, dass** die Biegeachse (80), um die die erste Energiekette (55) gebogen ist, senkrecht zu der Biegeachse (81) steht, um die die zweite Energiekette (56) gebogen ist.

13. Patientenpositioniereinrichtung nach einem der Ansprüche 1 bis 12,
**dadurch gekennzeichnet, dass** die Linearführung (3) deckenmontiert ist.

14. Verfahren zur Positionierung eines Patienten an einer Bestrahlungseinrichtung mittels einer Patientenpositioniereinrichtung, wobei die Patientenpositioniereinrichtung (1) einen Roboterarm (2) umfasst, der mindestens fünf Rotationsbewegungsachsen (16, 17, 18, 19, 20, 21) aufweist, wobei an dem Roboterarm (2) eine Patientenaufnahme gehalten ist, und wobei die Patientenpositioniereinrichtung mindestens eine Linearführung (3) umfasst, mittels der der Roboterarm (2) translatorisch verfahrbar ist, wobei das Verfahren die folgenden Schritte umfasst:
- Übernahme von Daten, die die Position eines Zielbereichs (71, 72) der Bestrahlungseinrichtung (4) angeben, von der Bestrahlungseinrichtung (4) und Ermitteln der Position eines zu bestrahlenden Bereichs (85) des Patienten (75) mittels einer bildgebenden Einheit und
- Bewegen der Patientenaufnahme derart, dass der zu bestrahlende Bereich (85) in dem Zielbereich (71, 72) der Bestrahlungseinrichtung angeordnet ist.

15. Verfahren nach Anspruch 14,
**dadurch gekennzeichnet, dass** die Bestrahlungseinrichtung (74) ortsfest angeordnet ist, und dass die Verfahrrichtung (70) der Linearführung (3) parallel zur Richtung des Strahls (7) der Bestrahlungseinrichtung (74) ausgerichtet ist.
